# FASCICULE DE BREVET EUROPEEN

(11) **EP 0 602 157 B1**
(45) Date de publication et mention de la délivrance du brevet: **16.01.2002**
(21) Numéro de dépôt: 92919440.5
(22) Date de dépôt: 03.09.1992
(51) Int. Cl.: C12N 15/51, A61K 31/70, C12N 9/00

(54) **STRUCTURE RIBOZYMIQUE DERIVEE DE L'ARN GENOMIQUE DU VIRUS DE L'HEPATITE DELTA**
RIBOZYMSTRUKTUR DIE VON DEN GENOMISCHEN RNS DES HEPATITIS DELTA VIRUS ABGELEITET IST
RIBOSOMAL STRUCTURE DERIVED FROM THE GENOMIC RNA STRUCTURE OF THE DELTA HEPATITUS VIRUS

(30) Priorité: 03.09.1991 FR 9110872
(43) Date de publication de la demande: 22.06.1994
(73) Titulaire: GENSET SA, F-75011 Paris (FR)
(72) Inventeur: BLUMENFELD, Marta, F-75013 Paris (FR); THILL, Gilbert, F-92270 Bois-Colombes (FR); VASSEUR, Marc, F-75013 Paris (FR)
(74) Mandataire: Warcoin, Jacques
(86) Numéro de dépôt international: FR9200840
(87) Numéro de publication internationale: WO9305157

(56) Documents cités:
- WO-A-91/04319
- WO-A-91/04324
- NATURE. vol. 350, 4 Avril 1991, LONDON GB pages 434 - 436 PERROTTA, A. & BEEN, M.D. 'A pseudoknot-like structure required for efficient self-cleavage of hepatitis delta virus RNA'
- NUCLEIC ACIDS RESEARCH. vol. 18, no. 23, 11 Décembre 1990, ARLINGTON, VIRGINIA US pages 6821 - 6827 PERROTTA, A. & BEEN, M.D. 'The self-cleaving domain from the genomic RNA of hepatitis delta virus: sequence requirements and the effect of denaturants'
- PROCEEDINGS OF THE NATIONAL ACADEMY OF SCIENCES OF USA. vol. 86, no. 6, Mars 1989, WASHINGTON US pages 1831 - 1835 WU, H.-N. ET AL. 'Human hepatitis delta virus RNA subfragments contain an autocleavage activity'
- JOURNAL OF VIROLOGY vol. 62, no. 12, Décembre 1988, BALTIMORE, US pages 4439 - 4444 KUO, M.Y.-P. ET AL. 'Characterization of self-cleaving RNA sequences on the genome and antigenome of human hepatitis delta virus'
- NUCLEIC ACIDS RESEARCH. vol. 19, no. 6, 25 Mars 1991, ARLINGTON, VIRGINIA US pages 1285 - 1289 SMITH, J.B. & DINTER-GOTTLIEB, G. 'Antigenomic hepatitis delta virus ribozymes self-cleave in 18 M formamide'
- NUCLEIC ACIDS RESEARCH vol. 19, no. 23, 11 Décembre 1991, ARLINGTON, VIRGINIA US pages 6519 - 6525 THILL, G. ET AL. 'Self-cleavage of a 71 nucleotide-long ribozyme derived from hepatitis delta virus genomic RNA'
- PROCEEDINGS OF THE NATIONAL ACADEMY OF SCIENCES OF USA vol. 88, no. 22, 15 Novembre 1991, WASHINGTON US pages 10163 - 10167 BRANCH, A.D. & ROBERTSON, H.D. 'Efficient trans cleavage and a common structural motif for the ribozymes of the human hepatitis delta agent'

## Description

La présente invention concerne une nouvelle structure ribozymique pouvant couper en trans une molécule cible d'ADN ou d'ARN.

Précisément, cette nouvelle structure ribozymique est une molécule d'ARN dérivée de l'ARN génomique du virus humain de l'hépatite delta.

L'ARN génomique du virus humain de l'hépatite delta présente des activités autocatalytiques. A partir de fragments d'ARN de taille réduite conservant une activité autbcatalytique de coupure, dérivés de l'ARN viral génomique, on a réussi selon l'invention à séparer de façon distincte une partie catalytique et une partie substrat, comme il sera explicité ci-après.

Dès lors, une coupure peut être obtenue au cours d'une réaction binaire en mélangeant la structure ribozymique ayant l'activité catalytique avec un ARN ou ADN substrat hétérologue. Cette caractérisation d'un fragment à activité catalytique de coupure permet, selon l'invention, d'utiliser ces structures ribozymiques de l'ARN génomique du virus de l'hépatite delta comme structure ribozymique du type "humain" pour le clivage d'ARN cibles, in vitro ou in vivo, à des fins expérimentales, thérapeutiques ou prophylactiques.

Les ribozymes sont des molécules d'ARN qui sont naturellement capables d'exercer des activités catalytiques, et particulièrement des activités RNAsiques. On a cherché, à partir de tels ribozymes naturels, à déterminer des structures ribozymiques artificielles capables d'exercer des coupures enzymatiques sur des ARN cibles spécifiques.

De tels ribozymes fournissent donc des outils thérapeutiques pouvant être utilisés dans le cadre d'approches de type oligonucléotides "antisens", notamment pour dégrader spécifiquement des ARN cibles de type pathogènes.

Les activités ribozymiques semblent largement répandues, et l'on a décrit des ARN catalytiques chez les bactéries, les protozoaires, les plantes, et les systèmes animaux. Les premières activités ribozymiques ont été mises en évidence tout d'abord dans les systèmes d'épissage des introns de classe I, puis dans la RNase P. Plus récemment, on a découvert que les génomes de nombreux virus à ARN, de plantes ou même de mammifères portaient également des activités ribozymiques qui intervenaient au cours du cycle de multiplication virale. Enfin, certains ARN transcrits à partir de régions d'ADN satellite chez le triton présentent également une activité ribozymique naturelle, servant à couper les transcrits concatémériques.

L'analyse de la structure des ribozymes génomiques de certains virus de plantes a mis en évidence des séquences réactionnelles communes ainsi que des structures secondaires consensus qui ont permis de construire des ribozymes pouvant agir sur mesure comme agents de clivage de séquences spécifiques d'ARN. L'analyse des régions impliquées dans ces coupures a fait apparaître une structure commune dite en "tête de marteau", ou en "T", nécessaire et suffisante pour effectuer la réaction nucléasique.

Une seconde classe de ribozymes, dérivés également d'un virus de plante présente une structure catalytique en "épingle à cheveux", ou structure en "L".

Ces différents types de ribozyme fonctionnent in vitro, mais les efficacités de coupure in vivo, dans des cellules animales ou humaines, sont encore peu efficaces. Cette inefficacité relative est vraisemblablement due, au moins en partie, au fait que toutes ces ribozymes sont dérivées de virus végétaux et non de virus animaux ou même humains.

Le virus de l'hépatite delta est un virus humain à ARN, que l'on trouve systématiquement associé au virus de l'hépatite B. L'ARN génomique, et également l'ARN anti-génomique de ce virus présentent tous deux une activité de clivage autocatalytique. La séquence du fragment d'ARN de longueur minimale présentant une activité ribozymique n'offre aucune ressemblance avec les motifs catalytiques déjà connus de type "T" ou "L". Le virus de l'hépatite delta se réplique dans les hépatocytes humains et son activité ribozymique fonctionne parfaitement dans ces cellules. Son activité ribozymique a été naturellement sélectionnée pour fonctionner dans des cellules humaines de foie et on dispose donc avec ce modèle d'un exemple parfait de ce que pourrait être un ribozyme actif chez l'homme.

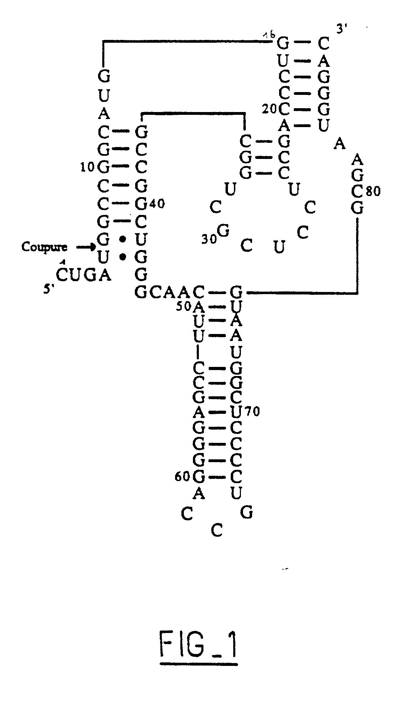

L'ARN génomique du virus de l'hépatite delta, long de 1700 nucléotides, comporte en effet une région capable d'exercer une coupure autocatalytique. Cette coupure autocatalytique peut être effectuée par un sous-fragment d'une longueur de 89 nucléotides dont la structure secondaire peut se schématiser sous la forme représentée à la figure 1.

Cette structure longue de 89 nucléotides comporte une conformation de type "pseudo-noeud". La coupure autocatalytique s'effectue au niveau de la liaison phosphodiester indiquée par la flèche sur la figure 1.

A partir de ce fragment ribozymique à activité autocatalytique on a, selon la présente invention, fourni des structures ribozymiques comportant une activité catalytique pouvant s'exercer en trans, au cours d'une réaction binaire, sur une seconde molécule d'ARN cible. Cette invention fournit de nouvelles molécules ribozymiques pouvant cliver efficacement des molécules d'ADN ou d'ARN cible, notamment de nature pathologique, à des fins thérapeutiques ou prophylactiques, dans des cellules humaines, animales ou même végétales.

Il s'agit là d'une nouvelle activité ribozymique en trans. La molécule responsable de cette coupure en trans présente une structure et une séquence ne correspondant à aucune autre structure ribozymique agissant en trans précédemment décrite.

En utilisant les techniques de synthèse chimique de l'ARN, des sous-fragments d'ARN correspondant à différentes régions de la molécule, représentée figure 1, ont été préparés. Ces différents fragments ont ensuite été testés dans des expériences de reconstructions afin de différencier les fragments substrats des fragments catalytiques.

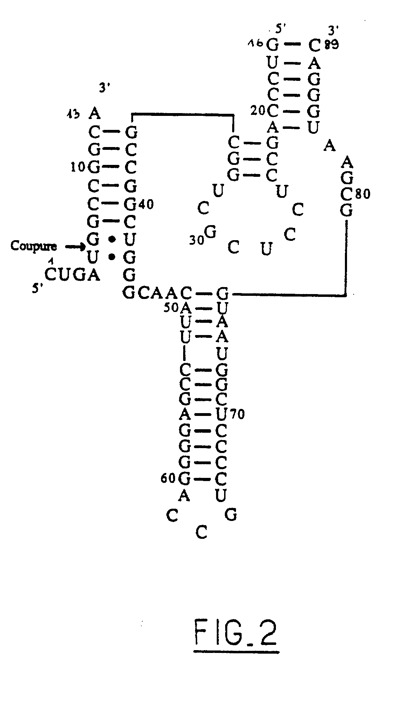

La présente invention a donc pour objet une structure ribozymique dérivée de l'ARN génomique du virus de l'hépatite delta consistant dans un ou plusieurs fragments issus de la séquence de 74 nucléotides comportant les nucléotides 16 à 89 représentés sur la Figure 2 , et capable d'exercer une activité catalytique de coupure en trans sur une autre molécule comportant une séquence spécifique d'acide nucléique ARN.

Par "autre molécule", on entend selon la présente demande que la coupure s'exerce sur une molécule autre que l'ARN génomique du virus de l'hépatite delta.

Dans un mode de réalisation particulièrement approprié la structure ribozymique est constituée de un ou deux fragment(s) issu(s) de la séquence de 74 nucléotides comportant les nucléotides de 16 à 89 représentés sur la figure 2.

Parmi les structures testées, les strutures ribozymiques préférées, c'est-à-dire les plus efficaces en termes d'activité de coupure, comportent un seul fragment.

En particulier, la structure ribozymique selon l'invention peut consister dans le fragment de 74 nucléotides comportant les nucléotides de 16 à 89 ou ce même fragment 16-89 dans lequel une partie seulement des nucléotides 50 à 77 ont été délétés. Lorsque la totalité de la séquence 50-77 a été délétée, le fragment perd de son activité catalytique de coupure.

Selon l'invention, ladite séquence d'acide nucléique de ladite autre molécule correspond à un fragment de l'ARN génomique du virus de l'hépatite delta substrat de ladite activité catalytique de coupure en trans.

En particulier, ladite séquence d'acide nucléique de ladite autre molécule substrat de l'activité catalytique pourra comporter la séquence 5'XGGCC3' avec X = C ou U, ladite structure ribozymique étant capable d'exercer l'activité nucléasique ou ribonucléasique sur la liaison phosphodiester entre X et G.

Dans un mode de réalisation, ladite séquence d'acides nucléiques de ladite autre molécule pourra comporter le fragment de 8 nucléotides allant des nucléotides 5 à 12 représentés sur la figure 2.

De façon appropriée, l'activité catalytique de coupure a lieu en présence d'ions métalliques divalents, notamment le magnésium.

Comme on l'a vu, la structure ribozymique selon l'invention exercera une activité ribonucléasique, notamment endoribonucléasique sur une autre molécule comportant un polyribonucléotide de séquence spécifique.

La structure ribozymique selon l'invention peut être utilisée à des fins thérapeutiques ou prophylactiques dans des cellules procaryotes ou eucaryotes, animales ou végétales, ladite autre molécule pouvant donc être une molécule hétérologue de cellules animales ou végétales.

On pourra utiliser la particule virale de l'hépatite delta comme système d'encapsidation ou tout autre vecteur pour la délivrance intra-cellulaire de structures ribozymiques selon l'invention, que ce soit dans des bactéries, des cellules eucaryotes, animales ou vététales.

D'autres avantages et caractéristiques de la présente invention apparaîtront à la lumière de la description détaillée qui va suivre. Cette description est faite en référence aux figures 1 à 4.
- La figure 1 représente la structure secondaire potentielle en "pseudo-noeud" d'un fragment autocatalytique de l'ARN génomique du virus de l'hépatite delta.
- La figure 2 représente la structure potentielle du complexe binaire de coupure en trans par un ribozyme dérivé du brin génomique du virus de l'hépatite delta.
- La figure 3 représente l'autoradiographie d'un gel de polyacrylamide 20 % sur lequel on a fait migrer les produits des incubations d'une structure ribozymique selon l'invention avec son substrat, après des temps variables.
- La figure 4 représente une analyse sur gel de polyacrylamide 10 %/7 M urée des produits d'une cinétique de coupure autocatalytique d'une structure ribozymique de 71 nucléotides de long comportant une délétion dans la structure auto-appariée 50-77.

### Exemple 1

Parmi les combinaisons testées on a découvert, comme on l'a vu, qu'il était possible d'effectuer une coupure en trans en utilisant comme substrat un fragment composé, par exemple, de 13 nucléotides (1 à 13) de la figure 2 et en utilisant comme structure ribozymique une molécule composée soit de deux fragments, comportant respectivement les nucléotides de 16 à 62 et 63 à 89, soit d'un seul fragment de 74 nucléotides de long comportant les nucléotides de 16 à 89.

Ce ribozyme de 74 nucléotides a été obtenu par transcription in vitro par la RNA polymérase T7 d'une matrice d'ADN bicaténaire. Cette matrice a été obtenue en synthétisant chimiquement les séquences d'ADN, en les hybridant, puis en amplifiant le produit obtenu par une réaction de PCR. Cette réaction de PCR permet, en utilisant les amorces appropriées, d'ajouter les séquences de promotions de la transcription permettant le fonctionnement de la polymérase T7.

La figure 2 illustre la structure ribozymique de 74 nucléotides et de son substrat de 13 nucléotides.

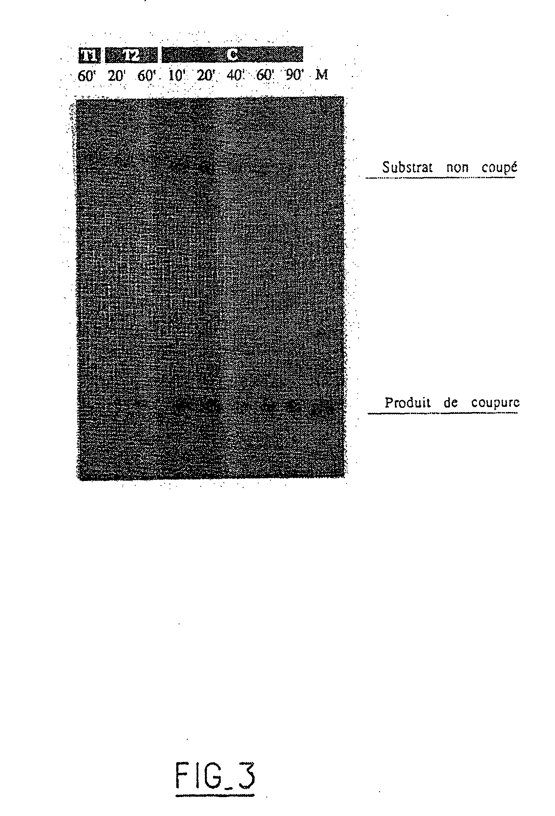

Ce ribozyme long de 74 nucléotides est capable d'effectuer une coupure rapide du substrat. Si l'on incube le ribozyme en présence du substrat pendant des temps variables de 0 à 60 minutes, dans un tampon contenant 10 mM MgCl₂, 50 mM Tris pH 8,0 2 mM spermidine, on observe (figure 3) qu'une coupure a lieu. La figure 3 représente l'autoradiographie d'un gel de polyacrylamide 20 % sur lequel on a fait migrer les produits des incubations du ribozyme avec son substrat après des temps variables. Le substrat a été marqué en 5' par du gamma-ATP ³²P par la T4 polynucléotide kinase et purifé sur gel de polyacrylamide avant utilisation. On observe qu'une partie importante de ce substrat est clivée dès les premières minutes de l'incubation, clivage matérialisé par une bande marquée longue de 5 nucléotides qui apparaît rapidement dans les conditions de fonctionnement ribozymique, c'est-à-dire en présence de magnésium. Les témoins effectués en présence d'EDTA sont négatifs, ce qui montre que l'on observe bien une réaction enzymatique dépendant de la présence de magnésium (ou d'autres ions divalents) ce qui est une caractéristique des ribozymes.

### Exemple 2

Les résultats expérimentaux ci-après montrent que la présence de la structure secondaire appariée située entre les nucléotides 50 et 77 n'est en partie pas indispensable à l'activité catalytique de ce ribozyme.

En partant de la structure d'un ribozyme de 89 nucléotides de long comme celui décrit dans la figure 1, on procède à des expériences de délétion de certaines régions, et en particulier des délétions de la structure en épingle à cheveux ("hairpin") comprise entre les nucléotides 50 et 77. En effet, cette "hairpin" est une structure secondaire extrêmement stable, que l'on retrouve dans toutes les configurations potentielles calculées par des programmes de minimalisation d'énergie appliqués à l'analyse de la structure de ce ribozyme et est probablement effectivement présente dans la structure active.

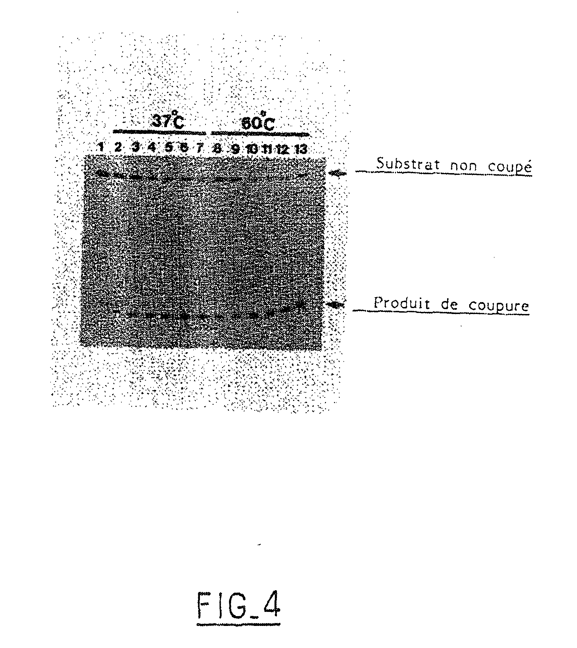

Afin de déterminer si cette structure secondaire appariée jouait un rôle catalytique ou bien structural, on a délété les nucléotides 53 à 61 et 66 à 74. Ces délétions ont été effectuées en synthétisant un fragment d'ADN comportant la séquence désirée, puis en amplifiant ce fragment par PCR comme décrit précédemment pour le ribozyme de 74 nucléotides. Après amplification avec les amorces appropriées et transcription par l'ARN polymérase de T7, on obtient une structure ribozymique longue de 71 nucléotides conservant son activité autocatalytique et fonctionnant à 37°C et à 60°C (figure 4).

Le ribozyme a été marqué en 5' par du gamma-ATP ³²P par la T4 polynucléotide kinase et purifié sur gel de polyacrylamide avant utilisation. On le dénature à 95°C pendant 1 minute en présence de 1 mM EDTA, puis il est refroidi rapidement à 0°C sur glace (snap-cool). Le milieu de réaction est ensuite ajusté en ajoutant 40 mM Tris pH 8,0 et 10 mM MgCl₂, puis incubé à 37°C (2 à 7) ou à 60°C (8 à 13). Des fractions aliquotes sont prélevées à des temps variables : 1 minute (2 & 8), 2 minutes (3 & 9), 5 minutes (4 à 10), 10 minutes (5 & 11), 20 minutes (6 & 12), et 60 minutes (7 & 13). Dans le contrôle (1), la réaction a été arrêtée au temps t 0 minute avant addition de 10 mM MgCl₂.

Le ribozyme natif (89 nucléotides de long) coupe son ARN avec un t 1/2 de 15 secondes, alors que le ribozyme délété présente un t 1/2 de 3 minutes. Donc, la délétion modifie la structure générale du ribozyme, ce qui modifie la cinétique de la réaction, mais elle n'affecte pas directement le site catalytique.

Il est donc possible de réduire la taille de la structure ribozymique fonctionnelle de la figure 2 en réduisant partiellement la longueur de cette boucle 50-77.

### LEGENDES RELATIVES A LA FIGURE 3 :

T1 = Substrat seul incubé dans le milieu de réaction pendant 60'
T2 = Substrat incubé dans le milieu de réaction en présence du ribozyme HΔV et de 10 mM EDTA
C = Cinétique de coupure = substrat incubé en présence du ribozyme HΔV
M = Marqueur de taille indiquant la position de la bande coupée attendue (5nt)

## Revendications

1. Structure ribozymique dérivée de l'ARN génomique du virus de l'hépatite delta, **caractérisée en ce qu'**elle consiste dans un ou plusieurs fragments issus de la séquence de 74 nucléotides comportant les nucléotides 16 à 89 représentés sur la Figure 2, ladite structure ribozymique étant capable d'exercer une activité catalytique de coupure en trans sur une autre molécule comportant une séquence spécifique d'acide nucléique d'ARN.

2. Structure ribozymique selon la revendication 1, **caractérisée en ce qu'**elle consiste dans le fragment de 74 nucléotides comportant les nucléotides 16 à 89 représentés sur la Figure 2.

3. Structure ribozymique selon la revendication 1, **caractérisée en ce qu'**elle consiste en deux fragments issus de la séquence de 74 nucléotides comportant les nucléotides 16 à 89 représentés sur la Figure 2.

4. Structure ribozymique selon la revendication 3, **caractérisée en ce qu'**elle est constituée de deux fragments, comportant respectivement les nucléotides 16 à 62 et 63 à 89 représentés sur la Figure 2.

5. Structure ribozymique selon la revendication 1, **caractérisée en ce qu'**elle consiste dans un fragment issu du fragment de 74 nucléotides constitué des nucléotides 16 à 89 de la Figure 2, fragment dans lequel une partie des nucléotides 50 à 77 ont été délétés.

6. Structure ribozymique selon la revendication 5, **caractérisée en ce que**, dans le fragment issu du fragment de 74 nucléotides constitué des nucléotides 16 à 89 de la Figure 2, les nucléotides 53 à 61 et 66 à 74 ont été délétés.

7. Structure ribozymique selon l'une des revendications 1 à 6, **caractérisée en ce que** ladite séquence d'acides nucléiques de ladite autre molécule correspond à un fragment de l'ARN génomique du virus de l'hépatite delta substrat de ladite activité catalytique de coupure en trans.

8. Structure ribozymique selon la revendication 7, **caractérisée en ce qu'**elle est capable d'exercer une activité catalytique de coupure sur la liaison phosphodiester entre X et G avec X = C ou U d'une séquence d'acide nucléique ARN comportant la séquence 5'XGGCC3'.

9. Structure ribozymique selon l'une des revendications 7 ou 8, **caractérisée en ce qu'**elle est capable d'exercer une activité catalytique de coupure sur une séquence d'acides nucléiques de ladite autre molécule comportant le fragment de 8 nucléotides allant des nucléotides 5 à 12 représentés sur la Figure 2.

10. Structure ribozymique selon l'une des revendications 1 a 10 pour utilisation comme agent thérapeutique ou prophylactique dans des cellules procaryotes ou eucaryotes, animales ou végétales.

11. Structure ribozymique capable d'exercer une activité catalytique de coupure en trans sur une autre molécule comportant une séquence spécifique d'acide nucléique d'ARN, ladite structure ribozymique étant **caractérisée en ce qu'**elle comprend un fragment issu du fragment de 74 nucléotides constitué des nucléotides 16 à 89 de la Figure 2, fragment dans lequel une partie des nucléotides 50 à 77 ont été délétés.

12. Structure ribozymique selon la revendication 12, **caractérisée en ce que**, dans le fragment issu du fragment de 74 nucléotides constitué des nucléotides 16 à 89 de la Figure 2, les nucléotides 53 à 61 et 66 à 74 ont été délétés.

## Patentansprüche

1. Von der genomischen RNA des Hepatitis-Delta-Virus abgeleitete Ribozymstruktur, **dadurch gekennzeichnet, daß** sie aus einem oder mehreren der 74 Nukleotide langen, aus den in Figur 2 dargestellten Nukleotiden 16 bis 89 bestehenden Fragmenten besteht, wobei diese Ribozymstruktur eine katalytische Aktivität, trans zu schneiden, auf ein anderes Molekül mit einer spezifischen RNA-Nukleinsäuresequenz auszuüben vermag.

2. Ribozymstruktur nach Anspruch 1, **dadurch gekennzeichnet, daß** sie aus dem 74-Nukleotidelangen Fragment, das die in Figur 2 dargestellten Nukleotide 16 bis 89 umfaßt, besteht.

3. Ribozymstruktur nach Anspruch 1, **dadurch gekennzeichnet, daß** sie aus zwei Fragmenten der 74-Nukleotide-langen Sequenz, die die in Figur 2 dargestellten Nukleotide 16 bis 89 umfaßt, besteht.

4. Ribozymstruktur nach Anspruch 3, **dadurch gekennzeichnet, daß** sie aus zwei Fragmenten, die die in Figur 2 dargestellten Nukleotide 16 bis 62 bzw. 63 bis 89 umfassen, besteht.

5. Ribozymstruktur nach Anspruch 1, **dadurch gekennzeichnet, daß** sie aus einem aus dem 74-Nukleotide-langen, aus den Nukleotiden 16 bis 89 der Figur 2 bestehenden Fragment stammenden Fragment besteht, in dem die Nukleotide 50 bis 77 teilweise deletiert worden sind.

6. Ribozymstruktur nach Anspruch 5, **dadurch gekennzeichnet, daß** in dem aus dem 74-Nukleotide-langen, aus den Nukleotiden 16 bis 89 der Figur 2 bestehenden Fragment stammenden Fragment die Nukleotide 53 bis 61 sowie 66 bis 74 deletiert worden sind.

7. Ribozymstruktur nach einem der Ansprüche 1 bis 6, **dadurch gekennzeichnet, daß** diese Nukleinsäuresequenz des anderen Moleküls einem genomischen RNA-Fragment des Hepatitis-Delta-Virus entspricht, das das Substrat der katalytischen Aktivität, trans zu schneiden, darstellt.

8. Ribozymstruktur nach Anspruch 7, **dadurch gekennzeichnet, daß** sie eine katalytische Aktivität, die Phosphodiesterbindung zwischen X und G, wobei X C oder U bedeutet, einer RNA-Nukleinsäuresequenz, welche die Sequenz 5'XGGCC3' umfaßt, zu schneiden, auszuüben vermag.

9. Ribozymstruktur nach einem der Ansprüche 7 oder 8, **dadurch gekennzeichnet, daß** sie eine katalytische Schneidaktivität auf eine Nukleinsäuresequenz dieses anderen Moleküls, welches das 8-Nukleotide-lange Fragment der in Figur 2 dargestellten Nukleotide 5 bis 12 umfaßt, auszuüben vermag.

10. Ribozymstruktur nach einem der Ansprüche 1 bis 9 zur Verwendung als Therapeutikum oder Prophylaktikum bei prokaryontischen oder eukaryontischen tierischen oder pflanzlichen Zellen.

11. Ribozymstruktur, die eine katalytische Aktivität, trans zu schneiden, auf ein anderes Molekül mit einer spezifischen RNA-Nukleinsäuresequenz auszuüben vermag, wobei diese Ribozymstruktur **dadurch gekennzeichnet ist, daß** sie ein aus dem 74-Nukleotide-langen, aus den Nukleotiden 16 bis 89 der Figur 2 bestehenden Fragment stammendes Fragment umfaßt, wobei bei dem Fragment die Nukleotide 50 bis 77 teilweise deletiert worden sind.

12. Ribozymstruktur nach Anspruch 11, **dadurch gekennzeichnet, daß** in dem aus dem 74-Nukleotide-langen, aus den Nukleotiden 16 bis 89 der Figur 2 bestehenden Fragment stammenden Fragment die Nukleotide 53 bis 61 sowie 66 bis 74 deletiert worden sind.

## Claims

1. Ribozyme structure derived from the genomic RNA of the hepatitis delta virus, **characterized in that** it consists of one or more fragments derived from the sequence of 74 nucleotides containing nucleotides 16 to 89 represented in Figure 2, the said ribozyme structure being capable of exerting a catalytic trans cleavage activity on another molecule containing a specific RNA nucleic acid sequence.

2. Ribozyme structure according to Claim 1, **characterized in that** it consists of the fragment of 74 nucleotides containing nucleotides 16 to 89 represented in Figure 2.

3. Ribozyme structure according to Claim 1, **characterized in that** it consists of two fragments derived from the sequence of 74 nucleotides containing nucleotides 16 to 89 represented in Figure 2.

4. Ribozyme structure according to Claim 3, **characterized in that** it consists of two fragments containing, respectively, nucleotides 16 to 62 and 63 to 89 represented in Figure 2.

5. Ribozyme structure according to Claim 1, **characterized in that** it consists of a fragment derived from the fragment of 74 nucleotides consisting of nucleotides 16 to 89 of Figure 2, from which fragment a portion of nucleotides 50 to 77 has been deleted.

6. Ribozyme structure according to Claim 5, **characterized in that** in the fragment derived from the fragment of 74 nucleotides consisting of nucleotides 16 to 89 of Figure 2, nucleotides 53 to 61 and 66 to 74 have been deleted.

7. Ribozyme structure according to one of Claims 1 to 6, **characterized in that** the said nucleic acid sequence of the said other molecule corresponds to a fragment of the genomic RNA of the hepatitis delta virus which is a substrate for the said catalytic trans cleavage activity.

8. Ribozyme structure according to Claim 7, **characterized in that** it is capable of exerting a catalytic cleavage activity on the phosphodiester bond between X and G with X = C or U of an RNA nucleic acid sequence containing the sequence 5'XGGCC3'.

9. Ribozyme structure according to either of Claims 7 and 8, **characterized in that** it is capable of exerting a catalytic cleavage activity on a nucleic acid sequence of the said other molecule comprising the fragment of 8 nucleotides ranging from nucleotides 5 to 12 represented in Figure 2.

10. Ribozyme structure according to one of Claims 1 to 10, for use as a therapeutic or prophylactic agent in prokaryotic or eukaryotic, animal or plant, cells.

11. Ribozyme structure capable of exerting a catalytic trans cleavage activity on another molecule containing a specific RNA nucleic acid sequence, the said ribozyme structure being **characterized in that** it comprises a fragment derived from the fragment of 74 nucleotides consisting of nucleotides 16 to 89 of Figure 2, from which fragment a portion of nucleotides 50 to 77 has been deleted.

12. Ribozyme structure according to Claim 12, **characterized in that** in the fragment derived from the fragment of 74 nucleotides consisting of nucleotides 16 to 89 of Figure 2, nucleotides 53 to 61 and 66 to 74 have been deleted.
